# EUROPEAN PATENT SPECIFICATION

(11) **EP 3 189 458 B1**
(45) Date of publication and mention of the grant of the patent: **07.10.2020**
(21) Application number: 15774976.3
(22) Date of filing: 27.08.2015
(51) Int. Cl.: G16B 25/00, G16B 45/00

(54) **METHODS AND STORAGE MEDIUM FOR VISUALIZING GENE EXPRESSION DATA**
VERFAHREN UND SPEICHERMEDIUM ZUR VISUALISIERUNG VON GENEXPRESSIONSDATEN
PROCÉDÉS ET SUPPORT D'INFORMATIONS POUR VISUALISER DES DONNÉES D'EXPRESSION DE GÈNE

(30) Priority: 05.09.2014 US 201462046550 P
(43) Date of publication of application: 12.07.2017
(73) Proprietor: Koninklijke Philips N.V., 5656 AG Eindhoven (NL)
(72) Inventor: MANKOVICH, Alexander Ryan, NL-5656 AE Eindhoven (NL); DIMITROVA, Nevenka, NL-5656 AE Eindhoven (NL); AGRAWAL, Vartika, NL-5656 AE Eindhoven (NL); BANERJEE, Nilanjana, NL-5656 AE Eindhoven (NL)
(74) Representative: van Iersel, Hannie
(86) International application number: PCT/IB2015/056486
(87) International publication number: WO 2016/034991

(56) References cited:
- RONALD VAN EIJK ET AL: "Visualization of regional gene expression biases by microarray data sorting", BIOTECHNIQUES, vol. 36, April 2004 (2004-04), pages 592-596, XP055229925,
- M. KANO ET AL: "Expression imbalance map: a new visualization method for detection of mRNA expression imbalance regions", PHYSIOLOGICAL GENOMICS, vol. 13, no. 1, 7 January 2003 (2003-01-07), pages 31-46, XP055229987, US ISSN: 1094-8341, DOI: 10.1152/physiolgenomics.00116.2002
- YAN ZHOU ET AL: "Genome-wide Identification of Chromosomal Regions of Increased Tumor Expression by Transcriptome Analysis", CANCER RESEARCH BIOINFORMATICS [ Z. Z.], AND BIOSTATISTICS [M. O.], vol. 63, 15 September 2003 (2003-09-15), pages 5781-5784, XP055229933,
- NILS GEHLENBORG ET AL: "Visualization of omics data for systems biology", NATURE METHODS, vol. 7, no. 3s, March 2010 (2010-03), pages S56-S68, XP55090459, ISSN: 1548-7091, DOI: 10.1038/nmeth.1436
- BO LI ET AL: "RSEM: accurate transcript quantification from RNA-Seq data with or without a reference genome", BMC BIOINFORMATICS, BIOMED CENTRAL, LONDON, GB, vol. 12, no. 1, 4 August 2011 (2011-08-04) , page 323, XP021104619, ISSN: 1471-2105, DOI: 10.1186/1471-2105-12-323

## Description

### FIELD

The invention relates generally to methods and systems for the analysis of gene expression profile data and, more specifically, to methods and systems for visualizing such data.

### BACKGROUND

The development and use of recombinant DNA and DNA sequencing technologies made it possible to collect and study the complete set of genes in a single cell, making it possible to identify genetic mutations associated with a particular cancer. DNA microarrays and RNA sequencing made it possible to study how those genes function to create gene products, making it possible to identify irregularities in gene expression that may be associated with a particular cancer. With this information, it may be possible to subtype particular cancers and identify the most effective course of treatment for a particular subtype of cancer.

For example, Sorlie *et. al.* established that new classifications of breast carcinoma could be made using gene expression profiles of known carcinoma subtypes tied to survival outcomes. Studying a set of 456 cDNA genes, the formerly-described Luminal/ER+ breast cancer subtype was broken down further into two or three possible subtypes: Luminal A, Luminal B, and Luminal C. Luminal A was found to have the highest expression of the ER α gene, GATA binding protein 3, X-box binding protein 1, trefoil factor 3, hepatocyte nuclear factor 3 α, and estrogen-regulated LIV-1; Luminal B and Luminal C both exhibited a low to moderate expression of genes specific to the Luminal subtype, with Luminal C (unlike Luminal B) expressing genes also expressed in basal-like and ERBB2+ breast carcinoma subtypes.

Sorlie and group later expanded on this study, finding that BRCA-1 mutated tumors all exhibited basal-like gene expression patterns. They also demonstrated that gene expression profiles could be used to classify the existing breast cancer subtypes across multiple independent datasets and subsequently correlated with clinical outcomes such as time to distant metastasis and overall survival.

Similarly, Pietenpol *et. al.* investigated the diversity of gene expression in the previously described Triple Negative Breast Cancer (TNBC) subtype. They obtained 587 TNBC cases from 21 breast cancer data sets and performed cluster analysis on the gene expression profiles of the cases. The cluster analysis identified 6 new subtypes based on their expression profiles: basal-like 1, basal-2, immunomodulatory, mesenchymal-like, mesenchymal stem-cell like, and luminal androgen receptor, as well as major signaling pathways affected in each subtype. TNBC cell lines were then screened to find matching expression profiles and the signaling pathways were pharmacologically targeted for treatment. This study serves as proof of concept that an informed investigation into expression signatures of known tumor subtypes can not only elucidate new subtypes but also advise more targeted treatment.

These types of transcriptome or exome studies, however, result in massive amounts of data that must be reviewed and analyzed in a way that is accurate, cost-effective, and timely. This is particularly true in the clinical context, where the resources and time available for the treatment of a single patient may not match the resources available for a comprehensive study. Accordingly, there is a need for improved methods and systems that enable the timely, accurate, and cost-effective evaluation of gene expression profile data.

Van Eijk et al. (BioTechniques 36:592-596, 2004) discloses visualisation of regional gene expression biases by microarray data sorting. Kano et al. (Physiol Genomics 13:31-46, 2003) discloses an expression imbalance map to detect mRNA expression imbalance regions.

### SUMMARY OF THE INVENTION

This summary is provided to introduce a selection of concepts in a simplified form that are further described below in the Detailed Description section. This summary is not intended to identify key features or essential features of the claimed subject matter, nor is it intended to be used as an aid in determining the scope of the claimed subject matter. The invention is defined by the scope of the appended claims.

Various embodiments of the present invention provide methods and products for visualizing transcriptomic and exomic data in a way that permits the comparison of different patient groups. These embodiments are suitable for many medical applications, including cancer diagnostics and treatment planning, particularly breast cancer.

In one aspect the present invention relates to a method for visualizing gene expression data. Gene expression data for at least one patient is organized into a plurality of windows of a specified size. An average RSEM score is calculated for all of the genes in each window, and the average RSEM scores for at least some of the windows is presented in a two-dimensional array, where one axis of the array organizes the windows by patient and the other axis of the array organizes the windows by sequence.

These and other features and advantages, which characterize the present non-limiting embodiments, will be apparent from a reading of the following detailed description and a review of the associated drawings. It is to be understood that both the foregoing general description and the following detailed description are explanatory only and are not restrictive of the non-limiting embodiments as claimed.

### BRIEF DESCRIPTION OF DRAWINGS

Non-limiting and non-exhaustive embodiments are described with reference to the following figures in which:
FIGS. 1A and 1B are a diagram of an exemplary method for gene expression visualization in accord with the present invention;
FIG. 2 is an example of a user interface for gene expression visualization generated by an embodiment of the present invention; in this example the interface presents 649 samples using 100kb windows, filtered to present the top 2% with most variance, stratified by menopausal status, age, and HER2/PR/ER receptor status;
FIG. 3 shows the interface of FIG. 2 with the interface adjusted to use a window of 23kb;
FIG. 4 is an example of a user interface for gene expression visualization generated by an embodiment of the present invention; in this example the interface presents 221 samples using 23kb windows, filtered to present the top 2% with most variance, stratified by menopausal status, age, HER2/PR/ER receptor status, and variants;
FIG. 5 shows the interface of FIG. 4 with the interface adjusted to use a window of 100kb;
FIG. 6 is an example of a user interface for gene expression visualization generated by an embodiment of the present invention; in this example the interface presents 593 samples using 100kb windows, filtered to present the top 2% with most variance, stratified by menopausal status, age, HER2/PR/ER receptor status, and PAM50 subtype calls;
FIG. 7 shows the interface of FIG. 6 with the interface adjusted to use a window of 23kb;
FIG. 8 is an example of a user interface for gene expression visualization generated by an embodiment of the present invention; in this example the interface presents 220 samples using 100kb windows, filtered to present the top 2% with most variance, stratified by menopausal status, age, HER2/PR/ER receptor status, PAM50 subtype calls, and variants;
FIG. 9 shows the interface of FIG. 8 with the interface adjusted to use a window of 23kb;
FIG. 10 is a block diagram of a system for gene expression visualization in accord with the present invention; and

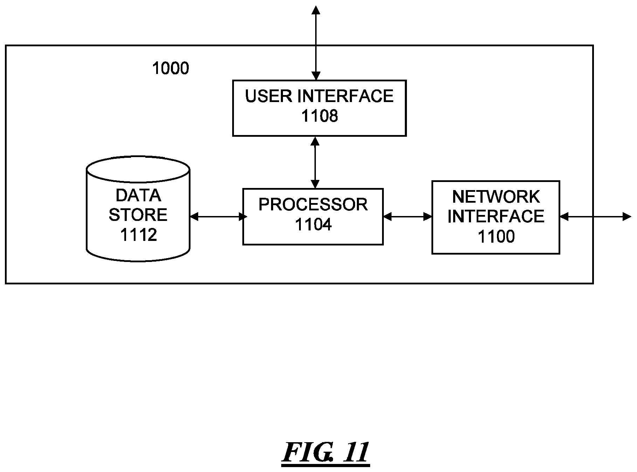

FIG. 11 presents the workstation 1000 of FIG. 10 in more detail.

In the drawings, like reference characters generally refer to corresponding parts throughout the different views. The drawings are not necessarily to scale, emphasis instead being placed on the principles and concepts of operation.

### DETAILED DESCRIPTION

Various embodiments are described more fully below with reference to the accompanying drawings, which form a part hereof, and which show specific exemplary embodiments. However, embodiments may be implemented in many different forms and should not be construed as limited to the embodiments set forth herein; rather, these embodiments are provided so that this disclosure will be thorough and complete, and will fully convey the scope of the embodiments to those skilled in the art. Embodiments may be practiced as methods, systems or devices. Accordingly, embodiments may take the form of a hardware implementation, an entirely software implementation or an implementation combining software and hardware aspects. The following detailed description is, therefore, not to be taken in a limiting sense.

Reference in the specification to "one embodiment" or to "an embodiment" means that a particular feature, structure, or characteristic described in connection with the embodiments is included in at least one embodiment of the invention. The appearances of the phrase "in one embodiment" in various places in the specification are not necessarily all referring to the same embodiment.

Some portions of the description that follow are presented in terms of symbolic representations of operations on non-transient signals stored within a computer memory. These descriptions and representations are the means used by those skilled in the data processing arts to most effectively convey the substance of their work to others skilled in the art. Such operations typically require physical manipulations of physical quantities. Usually, though not necessarily, these quantities take the form of electrical, magnetic or optical signals capable of being stored, transferred, combined, compared and otherwise manipulated. It is convenient at times, principally for reasons of common usage, to refer to these signals as bits, values, elements, symbols, characters, terms, numbers, or the like. Furthermore, it is also convenient at times, to refer to certain arrangements of steps requiring physical manipulations of physical quantities as modules or code devices, without loss of generality.

However, all of these and similar terms are to be associated with the appropriate physical quantities and are merely convenient labels applied to these quantities. Unless specifically stated otherwise as apparent from the following discussion, it is appreciated that throughout the description, discussions utilizing terms such as "processing" or "computing" or "calculating" or "determining" or "displaying" or "determining" or the like, refer to the action and processes of a computer system, or similar electronic computing device, that manipulates and transforms data represented as physical (electronic) quantities within the computer system memories or registers or other such information storage, transmission or display devices.

Certain aspects of the present invention include process steps and instructions that could be embodied in software, firmware or hardware, and when embodied in software, could be downloaded to reside on and be operated from different platforms used by a variety of operating systems.

The present disclosure also relates to an apparatus for performing the operations herein. This apparatus may be specially constructed for the required purposes, or it may comprise a general-purpose computer selectively activated or reconfigured by a computer program stored in the computer. Such a computer program may be stored in a computer readable storage medium, such as, but is not limited to, any type of disk including floppy disks, optical disks, CD-ROMs, magnetic-optical disks, read-only memories (ROMs), random access memories (RAMs), EPROMs, EEPROMs, magnetic or optical cards, application specific integrated circuits (ASICs), or any type of media suitable for storing electronic instructions, and each coupled to a computer system bus. Furthermore, the computers referred to in the specification may include a single processor or may be architectures employing multiple processor designs for increased computing capability.

The processes and displays presented herein are not inherently related to any particular computer or other apparatus. Various general-purpose systems may also be used with programs in accordance with the teachings herein, or it may prove convenient to construct more specialized apparatus to perform the required method steps. The required structure for a variety of these systems will appear from the description below. In addition, the present invention is not described with reference to any particular programming language. It will be appreciated that a variety of programming languages may be used to implement the teachings of the present invention as described herein, and any references below to specific languages are provided for disclosure of enablement and best mode of the present invention.

In addition, the language used in the specification has been principally selected for readability and instructional purposes, and may not have been selected to delineate or circumscribe the inventive subject matter. Accordingly, the disclosure of the present invention is intended to be illustrative, but not limiting, of the scope of the invention, which is set forth in the claims.

In brief overview, embodiments of the present invention transform massive amounts of genomic data into a visual form that is useful for analysts and clinicians. These embodiments and the associated representations can find application in various medical areas, including but not limited to cancer diagnostics, therapy planning, cancer subtyping, clinical decision support, etc. The visual representations of genomic data generated by embodiments of the present invention are capable of intuitively guiding the operator's choices, thus improving patient response while reducing overall cost and toxicities for the patient.

Genomics studies are fundamentally an exercise in comparison. An individual under consideration has her gene expression data compared against gene expression data taken from other individuals, healthy or otherwise. In other aspects, gene expression data taken from groups is reviewed to identify common features that may prove useful in diagnostics or treatment. A single set of expression data can include millions of bases, so studies of dozens or hundreds of such sets benefit from tools that make the analysis more accessible to an operator. While many methods exist today for gene expression analysis they typically focus on single genes and not on genomic regions that may get perturbed during tumorigenesis.

For simplicity, the following discussion assumes that the gene expression data (transcriptomic, exomic, etc.) is derived from breast carcinoma, although one of ordinary skill would understand that embodiments of the invention are not so limited. In fact, these embodiments are not only useful to breast cancer subtyping and clinical support, but to any analytics of carcinoma or gene expression data.

Cancer subtyping using gene expression is well established in breast cancer research. Individual breast cancer subtypes can often be characterized by copy number polymorphisms that affect large chromosomal regions, e.g., Luminal-A group tumors are sometimes associated with gain at 1q12-q41 and 16p12-p13.

That said, it is still unclear as to whether actual gene expression patterns are affected by genomic events affecting longer portions of the chromosomes. For that reason, embodiments of the present invention permit the imposition of arbitrary windows upon gene expression data under consideration to facilitate analysis. For simplicity, the following discussion assumes two discrete window sizes of 23kb and 100kb, although one of ordinary skill recognizes that the number of windows used in analysis as well as the actual window sizes themselves may vary in accord with the present invention and may depend upon such factors as the nature of the carcinoma studied.

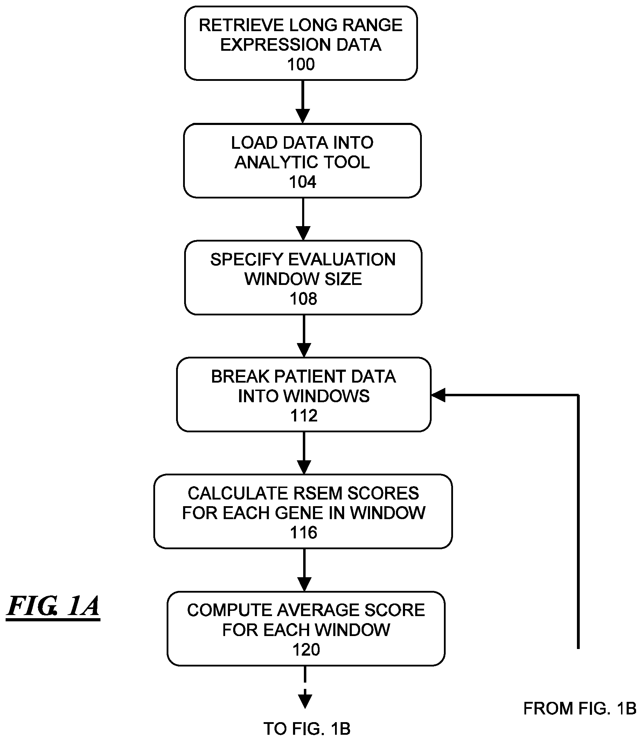

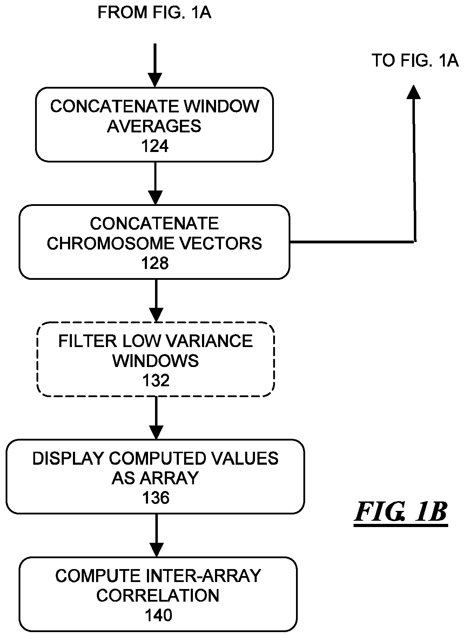

With reference to FIGS. 1A and 1B, in one embodiment the present invention begins by retrieving long range gene expression data for one or more patients (Step 100), such as TCGA-level 3 breast cancer gene expression data (RNA-Seq) generated at the Carolina Center for Genome Sciences, UNC at Chapel Hill, from at least one data source. The gene expression data from the at least one data source is loaded into an analytic tool (Step 104) such as R, available from the R Project for Statistical Computing at http://www.r-project.org/, for processing and subsequent display.

The analytic tool includes a graphical user interface element or other means for specifying the length of a window for evaluating the long range expression data (Step 108). The means is interactive, letting the operator adjust the evaluation window and the displayed representation substantially in real-time.

The defined evaluation window is repeatedly superimposed on the gene expression data for a particular patient, effectively converting the gene expression data into a series of concatenated sequences that are the size of the defined evaluation window (Step 112). The genes that fit into each window are identified and sample-specific gene scores for each gene are calculated (Step 116) using transcript abundance estimation software such as RSEM, available from the University of Wisconsin, Madison at http://deweylab.biostat.wisc.edu/rsem/README.html. The gene scores may be weighted according to how much they overlap with a particular window, i.e., weight multiplied by the sample gene score (not shown).

The RSEM scores for all of the genes within a window are averaged (Step 120); this is performed for each window-sized sequence in the gene expression data in serial or parallel, depending on the particular implementation of the embodiment. The averaged RSEM scores for all of the windows can be viewed together to form a larger chromosome-wide pattern (Step 124). These chromosome-wide vectors 108 are then concatenated to form a genome-wide long-range expression pattern 112 (Step 128). This process can be repeated seriatim for each patient's long range expression data or, in certain embodiments, this process is performed in parallel such that each patient's data is windowized, averaged, etc. substantially at the same time.

In certain embodiments, for the purpose of differentiating patient subgroups and identifying possible tumor driver regions in the long-range gene expression patterns, the variance for each window-sized sequence in the genome can be calculated and windows with low variance across patients can be excluded from presentation (Step 132). As discussed above, some embodiments of the present invention will include a graphical user interface element or another means for specifying a minimum level of variance for a particular window to be presented in an array format. The variance may be specified as an absolute value or a percentage, e.g., excluding 90%-98% of the windows with the least variance.

As depicted in later figures and discussed below, the computed values for the long-range gene expression data for each patient can be organized and presented as an array (Step 136) where a row in the array represents a patient and a column in the array represents a particular window-sized sequence in that patient's genome. All of the computed windows may be displayed, or they may be filtered for variance as discussed above.

In still other embodiments, inter-array correlation using, e.g., Pearson's r correlation, can be calculated for any given pair of samples in the matrix (Step 140). Hierarchical clustering of the results can be performed using, e.g., 1-IAC, as a distance metric, and enrichment of clinically meaningful subtypes can be evaluated using, e.g., a hypergeometric test.

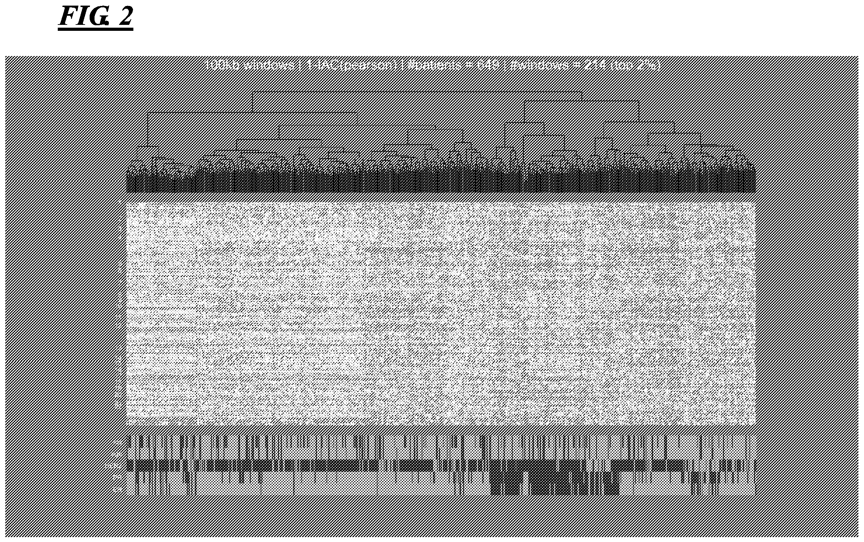

FIGS. 2-9 present examples of graphical output generated by embodiments of the invention. The most salient feature in the graphical output is the "heatmap" of the long range gene expression data, where each column in the array represents a patient and each row in the array represents a particular window-sized sequence in that patient's genome. All of the computed windows may be displayed, or they may be filtered for variance as discussed above.

The graphical output may also include a legend indicating the status of each patient with respect to various attributes of interest, such as age, HER2 status, TP53 status, etc. The legend may be placed above or below the heatmap (or to the left or right of the heatmap in embodiments where patients correspond to columns in heatmap), although in the following examples the legend is depicted consistently below the heatmap. The graphical output may also include a dendogram of sequence data as depicted in the following figures.

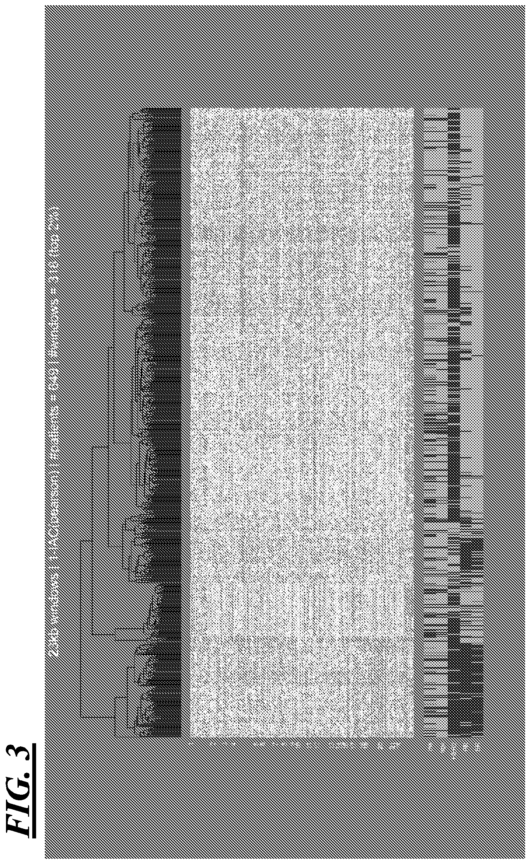

The examples in FIGS. 2 and 3 derive from a data set of long range expression patterns of 649 TNBC patients including ER, PR and HER2 status data. Simple hierarchical clustering reveals clear separation of triple negative breast cancer samples at any level of resolution, i.e., 23kb and 100kb in the available data set. While the 100kb resolution in FIG. 2 showed two distinct clusters, the 23kb resolution of FIG. 3 showed three distinct clusters. Interestingly, the hierarchical clustering of samples using the top 214 (2%) highly varying long 100kb regions revealed a cluster which contained 99 samples and was enriched with 75 TNBC samples (out of 102 TNBC in the entire set; p=1.7E-53).

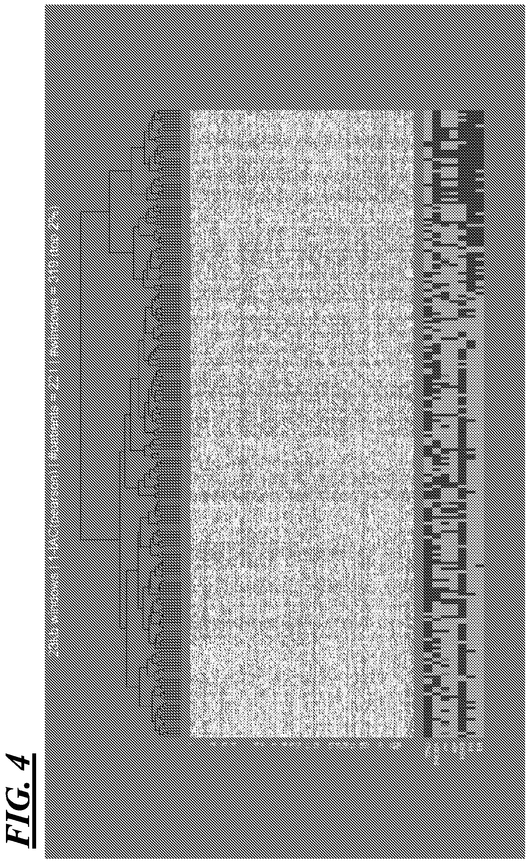

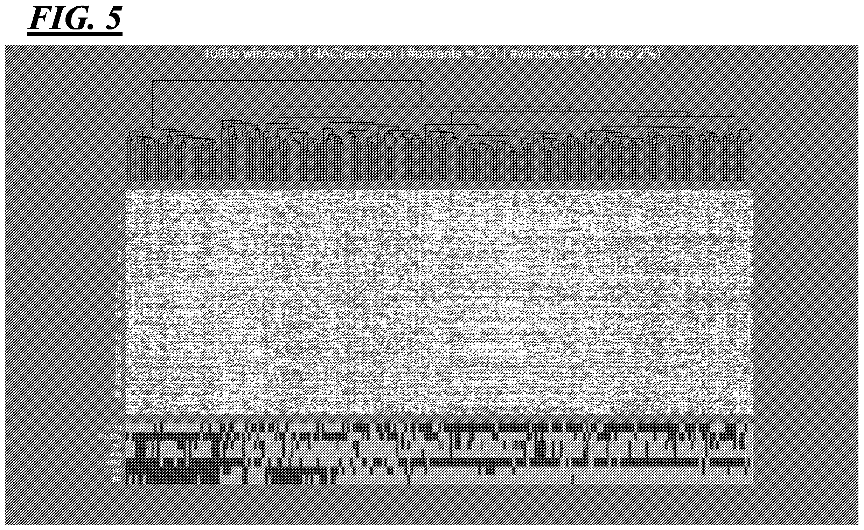

The examples in FIGS. 4 and 5 derive from a subset of the long range expression data of FIGS. 2 and 3 having 221 samples including ER, PR, Her2, age, menopausal status, PAM50 subtype information, p53 and PIK3CA mutation status. With reference to FIG. 4, the long range expression of 23Kb regions filtered to focus on the 2% of such regions having the highest variance segregated the samples into three different clusters: (1) *Cluster1*, a Luminal enriched cluster with 147 samples out of which 104 are ER+PR+Her2- samples (p-value <<0.01); (2) *Cluster2,* a Her2+ enriched cluster with 31 samples out of which 23 are Her2+ (p-value<<0. 01), and (3) *Cluster3,* a TNBC enriched cluster containing 33 samples out of which 26 are TNBC (p=1.5E-18); there are a total of 38 TNBC samples in the data set. Furthermore, in *Cluster3*, *28* of the samples had p53 mutations and 24 of those 28 were both TNBC and had p53 mutations (p-value <<0.05). In this TNBC cluster, there is enrichment of samples lacking PIK3CA mutations (n=31; p-value <<0.01). There was no clear association with age or menopausal status.

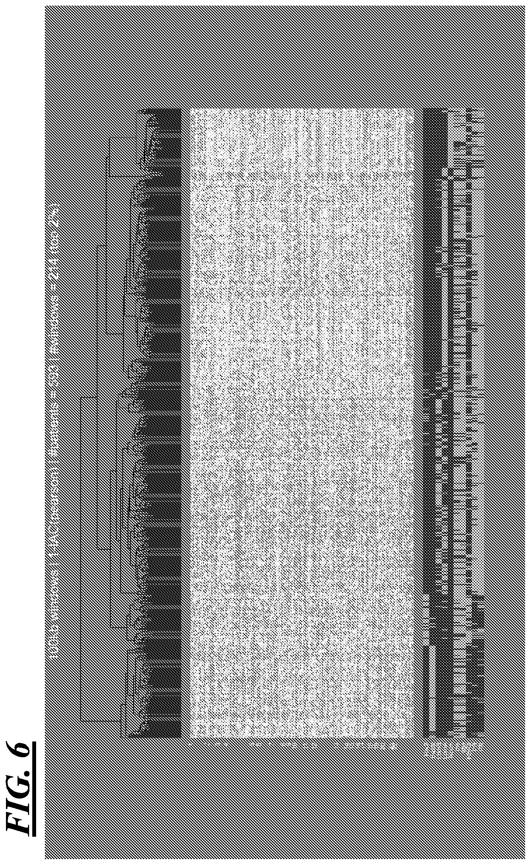

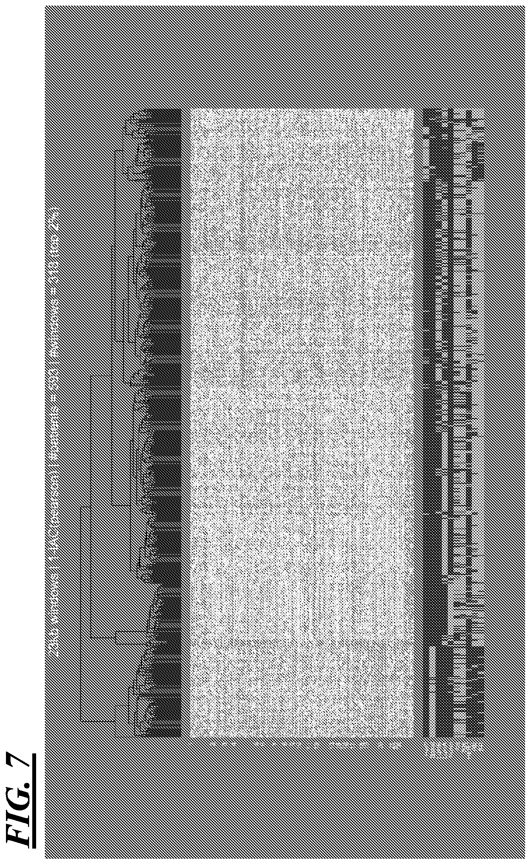

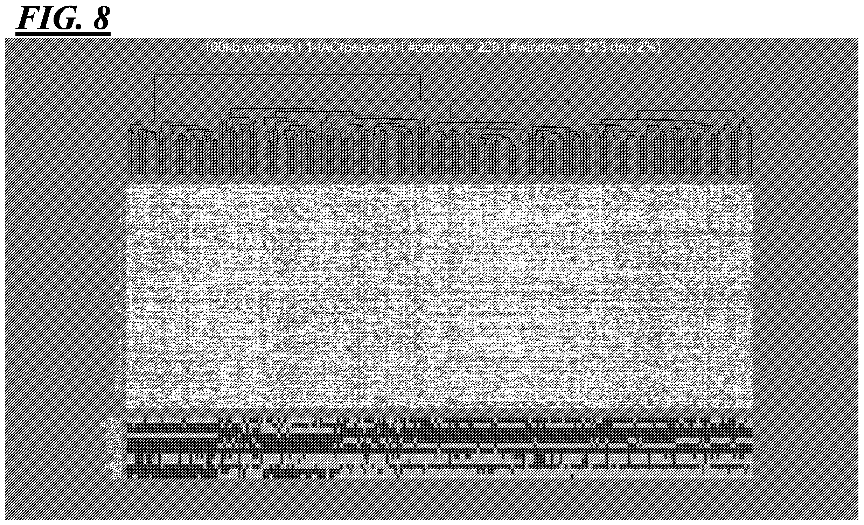

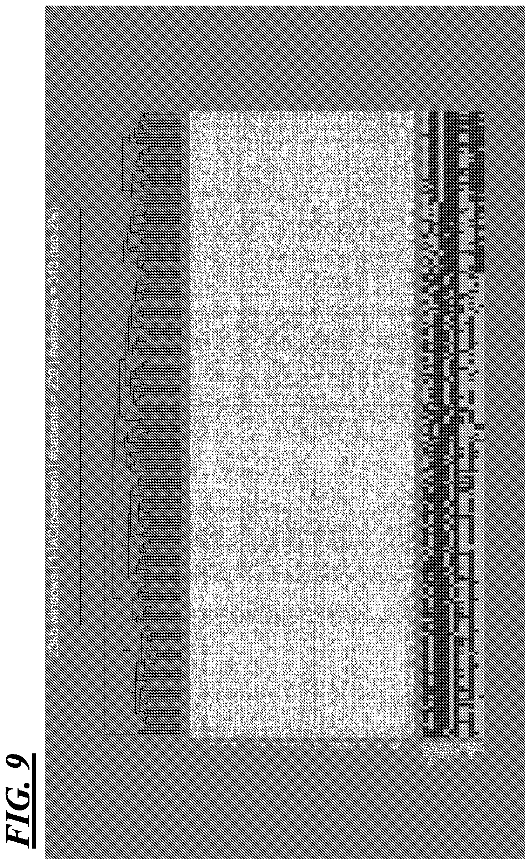

The examples in FIG. 6 and 7 derive from a subset of the long range expression data of FIGS. 2 and 3 having 593 samples with associated data for age, menopausal status, and ER, PR, HER2 status established by immunohistochemistry. With reference to FIG. 6, several distinct clusters displayed at a window resolution of 100k overlap with previously known subtypes: (1) *Cluster1*, i.e., basal subtype, which is closely related to TNBC, overlaps with the triple-receptor-negative cluster; (2) *Cluster2,* i.e., Her2+ subtype, defined by the hallmark HER2 positive status, overlaps with that feature cluster; (3) *Cluster3,* i.e., Luminal A/B subtype, as expected, has a higher proportion of HER2-positive samples, (4) *Cluster4,* i.e., Luminal A subtype, has few HER2-positive samples; and (5) *Cluster5*, i.e., Normal-like. It is of particular interest that there are two clusters of Luminal A, one which is isolated and appears as *Cluster4* and one which is mixed in with Luminal B patients and appears as *Cluster5*. With reference to FIG. 7, a similar pattern of separation is observed at a window resolution of 23kb resolution. This is conceivably another subtype not detected by PAM50 (such as Luminal C as described by Sørlie *et. al.*). Interestingly, overlap between these identified subtypes and PAM50 subtype calls was observed at window resolutions of both 23kb and 100kb, as illustrated in FIGS. 6 and 7. These subtypes also shared a similar association with PIK3CA and p53 mutations, as illustrated in FIGS. 8 and 9.

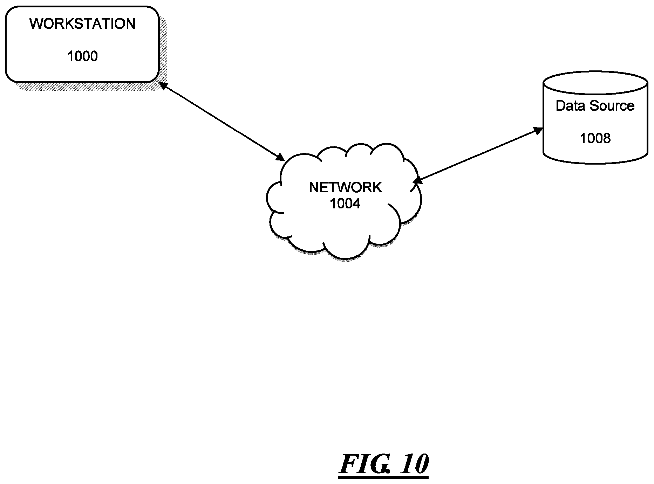

FIG. 10 depicts an exemplary embodiment of the present disclosure. A user operates a workstation 1000 such as a desktop computer or a laptop computer, although any device with a suitable interface and network connectivity such as a smartphone or tablet can be used. The workstation 1000 is in contact with a network 1004 such as the Internet or a wide-area network utilizing, e.g., a wired or wireless interface. The form of interface may vary depending on the particular nature of the workstation 1000. Typical interfaces include gigabit Ethernet, Wi-Fi (802.11a/b/g/n), and 3G/4G wireless interfaces such as GSM/WCDMA/LTE that enable data transmissions between workstation 1000 and other devices in communication with the network 104.

Like workstation 1000, data source 1008 is also in communication with the network 1004. In particular, various implementations of data source 1008 include physical machines such as a server computer, a blade server, clusters of servers, a virtual machine hosted by an on-demand computing service such as ELASTIC COMPUTE CLOUD a.k.a. EC2 offered by AMAZON.COM, INC. of Seattle, Washington, etc.

A user of a workstation 1000 communicates with data source 1008 through network 1004 to request long range expression data, which is subsequently processed by the workstation 1000 using, for example, a computer-based implementation of the method depicted in FIG. 1.

Figure 2 describes the workstation 1000 in additional detail. The network interface 1100 allows the workstation 1000 to receive communications from other devices and, in one embodiment, provides a bidirectional interface to the Internet. Suitable network interfaces 1100 include gigabit Ethernet, Wi-Fi (802.11a/b/g/n), and 3G/4G wireless interfaces such as GSM/WCDMA/LTE that enable data transmissions between workstation 1000 and other devices. A processor 1104 generates communications for transmission through the interface 1100 and processes communications received through the interface 1100 that originate outside the workstation 1000. A typical processor 1104 is an x86, x86-64, or ARMv7 processor, and the like. The user interface 1108 allows the workstation 1000 to receive commands from and provide feedback to an operator, for example, in connection with specification of a window size and/or a threshold for variability. Exemplary user interfaces include graphical displays, physical keyboards, virtual keyboards, etc. The data store 1112 provides both transient and persistent storage for data received via the interface 1100, data processed by the processor 1104, and data received or sent via the user interface 1108.

Embodiments of the present disclosure, for example, are described above with reference to block diagrams and/or operational illustrations of methods, systems, and computer program products according to embodiments of the present disclosure. The functions/acts noted in the blocks may occur out of the order as shown in any flowchart. For example, two blocks shown in succession may in fact be executed substantially concurrent or the blocks may sometimes be executed in the reverse order, depending upon the functionality/acts involved. Additionally, not all of the blocks shown in any flowchart need to be performed and/or executed. For example, if a given flowchart has five blocks containing functions/acts, it may be the case that only three of the five blocks are performed and/or executed. In this example, any of the three of the five blocks may be performed and/or executed.

The description and illustration of one or more embodiments provided in this application are not intended to limit or restrict the scope of the present disclosure as claimed in any way. The embodiments, examples, and details provided in this application are considered sufficient to convey possession and enable others to make and use the best mode of the claimed embodiments. The claimed embodiments should not be construed as being limited to any embodiment, example, or detail provided in this application. Regardless of whether shown and described in combination or separately, the various features (both structural and methodological) are intended to be selectively included or omitted to produce an embodiment with a particular set of features. Having been provided with the description and illustration of the present application, one skilled in the art may envision variations, modifications, and alternate embodiments falling within the broader aspects of the general inventive concept embodied in this application that do not depart from the broader scope of the claimed embodiments.

## Claims

1. A computer-implemented method for visualization and analysis of gene expression data, the method comprising:
receiving, from at least one data source, a digital file comprising one or more sets of long range gene expression data for one or more patients (step 100);
loading said long range gene expression data into an analytical tool comprising a user interface, wherein said user interface is configured to receive commands from and to provide feedback to an operator (step 104);
receiving via the user interface a specification of the size of at least one window for evaluating said long range gene expression data, wherein said window size is measured by sequence size in kb, and inputting said defined window size into said analytical tool (step 108);
converting the long range gene expression data for at least one patient into a series of concatenated windows that are the size of said defined window (step 112);
identifying each gene that is contained within each of said windows;
generating a sample-specific gene abundance score for each of said genes using transcript abundance estimation (step 116);
calculating an average gene abundance score for all of the genes identified in each of said windows (step 120); and
presenting the average gene abundance scores for at least some of the windows in a one- or two-dimensional array, wherein one axis of the array organizes the windows by patient and the other axis of the array organizes the windows by gene sequence.

2. The method of claim 1, wherein the transcript abundance estimation software is RSEM software.

3. The method of claim 2, wherein the averaging of the RSEM scores for all of the genes within a window is performed for each window-sized sequence in the long range gene expression data in series or in parallel.

4. The method of claim 2, wherein the averaged RSEM scores for all of the windows are presented together in the form of a larger chromosome-wide pattern, and chromosome-wide vectors are concatenated to form a genome-wide long range expression pattern.

5. The method of claim 2, wherein a minimum level of variance is specified by the operator through the user interface, and the variance for each window-sized sequence is calculated, and windows with low variance across patients are excluded.

6. The method of claim 2, further comprising the step of filtering out window-sized sequences of low variance.

7. The method of claim 5, wherein the series of concatenated windows for each patient are displayed together in an array to be evaluated by the operator.

8. The method of claim 7, wherein the series of concatenated windows for each patient are hierarchically clustered to form the array.

9. The method of claim 2, wherein the defined window size is 23kb or 100kb.

10. A computer-readable storage medium tangibly encoded with computer readable instructions, that when executed by a processor associated with a computing device, performs a method as per any one of claims 1-9.

## Patentansprüche

1. Eine auf einem Computer auszuführende Methode zum Visualisieren und Analysieren von Genexpressionsdaten, wobei die Methode folgende Schritte umfasst:
Abrufen einer digitalen Datei mit mindestens einem Genexpressionsdatensatz mit großer Reichweite für mindestens einen Patienten von mindestens einer Datenquelle (Schritt 100);
Laden des Genexpressionsdaten mit großer Reichweite in einem Analysewerkzeug mit einer Benutzerschnittstelle, die Befehle vom Bediener erhält und diesem Feedback bereitstellt (Schritt 104);
Abrufen eines Werts für mindestens eine Fenstergröße über die Benutzerschnittstelle, um die Genexpressionsdaten mit großer Reichweite auszuwerten, wobei die Fenstergröße als Sequenzgröße in KB gemessen wird, sowie Eingeben der definierten Fenstergröße in das Analysewerkzeug (Schritt 108);
Konvertieren der Genexpressionsdaten mit großer Reichweite für mindestens einen Patienten in eine Reihe verketteter Fenster, die die Größe des definierten Fensters aufweisen (Schritt 112);
Ermitteln aller in den einzelnen Fenstern enthaltenen Gene;
Generieren eines probenspezifischen Genbestandswerts für die einzelnen Gene mithilfe einer Transkript-Mengenschätzung (Schritt 116);
Berechnen eines durchschnittlichen Genbestandswerts für alle für die einzelnen Fenster ermittelten Gene (Schritt 120); und
Darstellen der durchschnittlichen Genbestandswerte für mindestens einige der Fenster in einer ein- oder zweidimensionalen Anordnung, wobei eine Achse der Anordnung die Fenster nach Patienten und die andere Achse die Fenster nach Gensequenz organisiert.

2. Die Methode gemäß Anspruch 1,
wobei es sich bei der Software für die Transkript-Mengenschätzung um RSEM-Software handelt.

3. Die Methode gemäß Anspruch 2,
wobei die Mittelung der RSEM-Werte aller Gene in einem Fenster für die einzelnen Sequenzen mit Fenstergröße in den Genexpressionsdaten mit großer Reichweite in Reihe oder parallel erfolgt.

4. Die Methode gemäß Anspruch 2,
wobei die gemittelten RSEM-Werte für die Fenster gemeinsam in Form eines übergeordneten Chromosomweiten Musters dargestellt werden. Hierbei sind die Chromosome-weiten Vektoren verkettet, um ein Genomweites Expressionsmuster mit großer Reichweite zu bilden.

5. Die Methode gemäß Anspruch 2,
wobei der Benutzer über die Benutzerschnittstelle ein Mindestmaß an Varianz angibt. Hierbei wird die Varianz für die einzelnen Sequenzen mit Fenstergröße berechnet, um Fenster mit geringer patientenübergreifender Varianz auszuschließen.

6. Die Methode gemäß Anspruch 2,
die zudem das Herausfiltern von Sequenzen in Fenstergröße mit geringer Varianz umfasst.

7. Die Methode gemäß Anspruch 5,
wobei die Reihe von verketteten Fenstern für die einzelnen Patienten gemeinsam in einer Anordnung angezeigt werden, um vom Benutzer ausgewertet werden zu können.

8. Die Methode gemäß Anspruch 7,
wobei die Reihe von verketteten Fenstern für die einzelnen Patienten hierarchisch gruppiert sind, um eine Anordnung zu bilden.

9. Die Methode gemäß Anspruch 2,
wobei die definierte Fenstergröße 23 KB oder 100 KB beträgt.

10. Ein computerlesbares Speichermedium, das konkret mit von einem Computer lesbaren Anweisungen codiert ist, die beim Ausführen auf einem Computer-Prozessor die Methode gemäß einer der Ansprüche 1 bis 9 ausführen.

## Revendications

1. Procédé mis en œuvre par ordinateur pour visualiser et analyser des données d'expression de gène, le procédé comprenant :
la réception, depuis au moins une source de données, d'un fichier numérique comprenant un ou plusieurs ensembles de données d'expression de gène à longue portée pour un ou plusieurs patients (étape 100) ;
le chargement des dites données d'expression de gène à longue portée dans un outil analytique comprenant une interface utilisateur, dans lequel ladite interface utilisateur est configurée pour recevoir des commandes d'un opérateur et lui transmettre des commentaires (étape 104) ;
la réception via l'interface utilisateur d'une spécification de la taille d'au moins une fenêtre pour évaluer lesdites données d'expression de gène à longue portée, dans laquelle ladite taille de fenêtre est mesurée par la taille de la séquence en kb, et pour saisir ladite taille de fenêtre définie dans ledit outil analytique (étape 108) ;
la conversion des données d'expression de gène à longue portée pour au moins un patient dans une série de fenêtres concaténées à la taille de ladite fenêtre définie (étape 112) ;
l'identification de chaque gène contenu à l'intérieur de chacune desdites fenêtres,
la génération d'un score d'abondance de gène spécifique de l'échantillon pour chacun desdits gènes utilisant l'estimation d'abondance de transcription (étape 116) ;
le calcul d'un score d'abondance moyen de gène pour tous les gènes identifiés dans chacune desdites fenêtres (étape 120) ; et
la présentation des scores d'abondance moyens de gènes pour au moins certaines des fenêtres dans un réseau unidimensionnel ou bidimensionnel, dans lequel un axe du réseau organise les fenêtres par patient et l'autre axe du réseau organise les fenêtres par séquence de gène.

2. Procédé selon la revendication 1, dans lequel le logiciel d'estimation d'abondance de transcription est le logiciel RSEM.

3. Procédé selon la revendication 2, dans lequel la moyenne des scores RSEM pour tous les gènes à l'intérieur d'une fenêtre est réalisée pour chaque séquence grandeur-fenêtre dans les données d'expression de gène à longue portée en série ou en parallèle.

4. Procédé selon la revendication 2, dans lequel les scores RSEM moyens pour toutes les fenêtres sont présentés ensemble sous la forme d'un plus grand modèle de la taille des chromosomes, et les vecteurs de la taille des chromosomes sont concaténés pour former un modèle d'expression à longue portée de la taille du génome.

5. Procédé selon la revendication 2, dans lequel un niveau minimum de variance est spécifié par l'opérateur à travers l'interface utilisateur, et la variance pour chaque séquence grandeur-fenêtre est calculée, et les fenêtres avec une faible variance parmi les patients sont exclues.

6. Procédé selon la revendication 2, comprenant en outre l'étape de filtrage des séquences grandeur-fenêtre de faible variance.

7. Procédé selon la revendication 5, dans lequel les séries de fenêtres concaténées pour chaque patient sont affichées ensemble dans un réseau pour être évaluées par l'opérateur.

8. Procédé selon la revendication 7, dans lequel les séries de fenêtres concaténées pour chaque patient sont hiérarchiquement regroupées pour former un réseau.

9. Procédé selon la revendication 2, dans lequel la taille de fenêtre définie est 23kb ou 100kb.

10. Support de stockage lisible par ordinateur concrètement codé avec les instructions lisibles par ordinateur qui lorsqu'exécutées par un processeur associé à un dispositif informatique, réalise un procédé selon l'une quelconque des revendications 1 à 9.
